## Europäisches Patentamt

## European Patent Office

## Office européen des brevets

(19)

(11) Veröffentlichungsnummer: **0 049 341 B1**

(12) **EUROPÄISCHE PATENTSCHRIFT**

(45) Veröffentlichungstag der Patentschrift:
**19.02.86**

(21) Anmeldenummer: **81106054.0**

(22) Anmeldetag: **01.08.81**

(51) Int. Cl.⁴: **C 12 N 5/00,** A 61 F 2/00, A 01 N 1/02

(54) **Verfahren zur Vorbereitung von alloplastischen Implantationen und Organtransplantationen.**

(30) Priorität: **21.07.81 EP 81105731**
**07.10.80 EP 80106066**

(43) Veröffentlichungstag der Anmeldung:
**14.04.82 Patentblatt 82/15**

(45) Bekanntmachung des Hinweises auf die Patenterteilung:
**19.02.86 Patentblatt 86/8**

(84) Benannte Vertragsstaaten:
**AT BE CH DE FR GB IT LI LU NL SE**

(56) Entgegenhaltungen:
**WO - A - 80/01350**

**NATURE, Band 227, 29. August 1970 R.Y. CALNE et al.:
"Immunosuppressive Effects of Soluble Cell Membrane
Fractions, Donor Blood and Serum on Renal Allograft
Survival" Seiten 903-906**

(73) Patentinhaber: **Theurer, Karl Eugen, Prof.Dr.med.,
Brunnwiesenstrasse 23, D-7302 Ostfildern 1 (DE)**
Patentinhaber: **Theurer, Karl Georg, Dr.med.,
Brunnwiesenstrasse 23, D-7302 Ostfildern 1 (DE)**

(72) Erfinder: **Theurer, Karl Eugen, Prof.Dr.med.,
Brunnwiesenstrasse 23, D-7302 Ostfildern 1 (DE)**
Erfinder: **Theurer, Karl Georg, Dr.med.,
Brunnwiesenstrasse 23, D-7302 Ostfildern 1 (DE)**

## Beschreibung

Die Erfindung betrifft die Vorbereitung von Implantationen von alloplastischen oder devitalisierten biologischen Materialien, wie auch von allogenen und möglicherweise xenogenen Organtransplantaten vor der Implantation bzw. Transplantation. Es wird dabei auf der Oberfläche des Implantates bzw. Transplantates, die mit Zellgewebe oder Knochen des Empfängerorganismus in Kontakt kommen soll, ein Zellrasen von in Kultur gezüchteten lebenden Gewebezellen nach den bekannten Methoden der Zellkultur aufgezüchtet und zum Auf- bzw. Einwachsen, auch in Krypten oder Poren des alloplastischen Materials gebracht. Dabei können Zellen verwendet werden, die aus autologen Explantaten vom späteren Empfänger oder von allogenen, xenogenen bzw. foetalen Spendern kultiviert werden.

Zur Erzielung der Gewebeverträglichkeit mit dem späteren Empfängerorganismus müssen allogene und xenogene Gewebe während der Kultivierung an den Empfängerorganismus adaptiert werden. Foetale Zellen benötigen diese Konditionierung nicht. Andererseits muss im Empfängerorganismus durch wiederholte Vorbehandlung mit den entsprechenden Zellen oder daraus hergestellten Zellextrakten bzw. dem Medium der Zell- bzw. Gewebekulturen eine immunologische Toleranz erzeugt werden.

Aufgezüchtete Zellen auf alloplastischen Materialien und Gewebetransplantaten beschleunigen das Einheilen und verbessern die Verankerung im Wundbett durch Neubildung von Zellen, insbesondere von Bindegewebsfibrillen und Interzellularsubstanz. Dadurch kommt eine weitgehend physiologische Verankerung des Implantates zustande. Die Beschichtung mit lebenden Zellen ist aber auch für Organtransplantate ein Vorteil, weil die aufgezüchteten Zellen eine höhere Zellteilungsrate besitzen als diejenigen der Organoberfläche und bei Verwendung von allogenen oder xenogenen Spendern durch eine konditionierende Vorbehandlung des Empfängers mit den Histokompatibilitätsfaktoren der aufgezüchteten Zellen und möglichst auch des Organtransplantates eine immunologische Abstossungsreaktion vermieden wird. Bei Implantationen und Transplantationen, die ohne vorherige Aufzüchtung von Zellrasen erfolgen, ist in vivo das Aufwachsen von Zellen erschwert, weil im Wundbett die Zellen im Gewebe gebunden sind und nicht frei vorkommen. Die Aufzüchtung eines Geweberasens in vitro durch Beimpfung mit Zellsuspensionen wird durch die grössere Oberfläche der isolierten Zellen begünstigt. Die Kultivierung der Zellen kann schon längere Zeit vor der Organverpflanzung auch aus Gewebeexplantaten oder aus leukozytären Blutzellen des Organspenders erfolgen. Bei Verwendung allogener oder xenogener Zellen ist eine wechselseitige Konditionierung vom Empfänger und den zu übertragenden Spenderzellen und -geweben möglich. Die übertragenen Zellen übernehmen eine Vermittlerfunktion zum Empfängerorganismus.

Als alloplastische Materialien werden vorwiegend Kunststoffe, Keramik, Biogläser und Metalllegierungen, auch in Kombination verwendet. Als Implantate dienen auch devitalisierte biologische Materialien z.B. aus Knochen, Knochenspänen und -splittern sowie Zähnen, Sehnen, Hornhäuten, Eihäuten und Nabelschnur. Diese werden zur Rekonstruktion von Gewebedefekten an Knochen, Sehnen, Blutgefässen, Haut, serösen Häuten, sowie zum Ersatz von Gelenken als Endoprothesen wie auch von Sehnen verwendet. Bei der Implantation von Zähnen und Zahnersatz ist der Faserverbund zwischen Implantat und Knochen nicht nur zur Fixierung sondern auch wegen des epithelialen Abschlusses nach aussen zur Mundhöhle, zur Vermeidung von Infektionen und der Bildung von infektiösen Herden im Gebiet des Zahnersatzes wichtig.

Die Erfindung beinhaltet auch die Verwendung von Bioklebern, die für Körperflüssigkeiten diffusibel sind, wie z.B. Fibrin, das aus einer Mischung von Fibrinogen und Thrombin entsteht oder verflüssigtes Kollagen oder Elastin mit einem Verfestiger. Es werden dabei Gewebe- oder Zellextrakte aus den kultivierten Zellen oder anderen wachstumsstimulierenden Faktoren, wie sie insbesondere nach EPA Nr. 80 106 066.6 gewonnen werden, dem wässrigen Lösungsmittel des Bioklebers zugesetzt. Es können auch isolierte Zellen aus der Kultur in den Kleber eingebracht werden. Diese werden dann bei der Retraktion des Fibrins zusammengepresst und daraus Zellinhaltsstoffe in Freiheit gesetzt, welche die bindegewebige Organisation des Bioklebers durch Ersatz mit körpereigenen Geweben beschleunigt.

Für die Einpflanzung alloplastischer Materialien bietet eine Mikroporosität oder netz- bzw. schwammartig durchbrochene Struktur der Oberfläche für die feste Verankerung des Implantates Vorteile. Metalle, auf denen Zellen nicht spontan anwachsen, werden mit dafür geeigneten Materialien, z.B. aus inertem Kunststoff, Biogläsern, Porzellan oder aber mit Fibrin, Kollagen oder Elastin vor der Beimpfung mit Zellen beschichtet. Die Implantation von solchen Endoprothesen erfordert keinen Kunststoff-«Zement». Die Erfindung betrifft jedoch die Aufzüchtung eines festgewachsenen Zellrasens und nicht die dazu verwendeten Materialien.

Die Zellkulturen werden aus Gewebeproben, die chirurgisch oder durch Biopsie von einem Spender gewonnen werden, nach bekannten Methoden der Zell- und Gewebezüchtung angelegt (vgl. Brigitte Mauersberger: Aktuelle Probleme der Zellzüchtung: Gustav Fischer Verlag Stuttgart, 1971; Karl Friedrich Bauer: Methodik der Zell- und Gewebezüchtung: S. Hirzel Verlag Stuttgart, 1974). Bei Zähnen können Stanzzylinder aus der vorgesehenen Implantationsstelle oder extrahierte Zähne für die Anzüchtung der Zellkulturen verwendet werden. Durch Klonen können verschiedene Zellarten gezüchtet werden, wie z.B. Osteoblasten, Odontoblasten, Chondroblasten, Myoblasten, Fibroblasten, epitheliale Zellen und andere. Die gewonnenen Zellinien können dann in Massenzüchtung weiter kultiviert werden. Es ist zweckmässig zur Beschichtung von Implantaten bzw. Transplantaten die Zellarten zu verwenden, die zum Anwachsen im Wundbett besonders geeignet sind, also vorzugsweise Fibroblasten. Diese lassen sich auch besonders leicht züchten, weil sie eine grosse Zellteilungspotenz besitzen. Es können auch heteroploide Zellen ohne onkogene Potenz Verwendung finden. Die explantierten Zellen werden gegebenenfalls in wiederholten Passagen gezüchtet und dabei mit Wirkfaktoren aus Organextrakten stimuliert, z.B. aus Leber, Plazenta, Thymus, foetalem Bindegewebe, aus Nabelschnur oder anderen foetalen Geweben (vgl. EPA 80 106 066.6; Paffenholz V. und K. Theurer: Einfluss von makromolekularen Organsubstanzen auf menschliche Zellen in vitro: Der Kassenarzt 27, Sept. 1978).

Zur Beimpfung des sterilen Implantates werden durch enzymatische Ablösung der Zellen von der Unterlage Zellsuspensionen gewonnen. Diese werden auf das Implantat oder Transplantat an verschiedenen Stellen aufgeimpft und dann dauernd oder rhythmisch mit geeigneten Nährmedien nach bewährten Methoden der Zellzüchtung befeuchtet. Jede mechanische Alteration der Zellen muss dabei vermieden werden.

Zur Adaptation an den späteren Empfänger werden allogene oder xenogene Gewebe mit entsprechenden Extrakten aus Organgeweben oder aus Blutzellen bzw. -serum des späteren Empfängers in einem Minimalnährmedium mindestens während 4 Stunden behandelt. Dies kann unmittelbar vor der Implantation geschehen. Die Protein- oder Peptidkonzentration der verwendeten Organextrakte liegt zwischen mg und ng/ml Nährmedium.

Zur Vorbehandlung des Empfängers von mit allogenen oder xenogenen Zellen besetzten Implantaten oder Organtransplantaten wird dieser mindestens 2 Tage vor der Implantation mit steigenden Zellzahlen oder Konzentrationen des eiweisshaltigen Extraktes und bzw. oder den dekantierten Kulturmedien der entsprechenden Zellkulturen behandelt. Diese Vorbehandlung entspricht der Erzeugung einer Low-zone Toleranz.

Die Toleranzerzeugung wird gefördert durch die Anwendung einer Mischung von nativen Molekülen und ihren Untereinheiten bzw. Bestandteilen. Es können dazu auch sulfatierte und phosphatierte Trockenextrakte der Gewebe bzw. der gezüchteten Gewebezellen parenteral wiederholt injiziert wie auch in steigenden Konzentrationen oral bzw. enteral gegebenenfalls in Liposomen oder in der wässrigen Phase einer Wasser-in-Öl-Emulsion in dünndarmlöslichen Kapseln angewandt werden. Bei der parenteralen Injektion werden ansteigende Konzentrationen vom pg- bis mg-Bereich in sich verlängernden Intervallen von Stunden bis 2–3 Tagen angewandt. Es können gezüchtete Zellen aus Eihaut und Nabelstrang wie auch aus anderen Körperbereichen, insbesondere aus Haut, Plazenta, Thymus, Blutadern oder Zahnleiste verwendet werden. Zur immunologisch tolerogenen Vorbehandlung des Empfängers lassen sich auch gezüchtete Zellen, wie auch vom Spender gewonnenen Zellen oder Gewebeextrakte verwenden. Nichtkompatible Faktoren können auch aus dekantierten Kulturmedien, in denen die Vitalkonservierung stattfindet, gewonnen werden. Zur Vorbereitung von allogenen bzw. xenogenen Organtransplantaten werden nichtkompatible Faktoren aus dem Spenderorganismus des Transplantates, auch in Form von gezüchteten Zellen oder eiweiss- bzw. peptidhaltigen Organextrakten zur Vorbehandlung des Empfängers verwendet.

Das Verfahren von O'Connor, N. et al. (Dr. H. Green) (Lancet 1, 75, 1981) über die Abdeckung von Verbrennungen mit in Kultur gezüchtetem Epithel unterscheidet sich grundsätzlich von vorliegendem Verfahren durch die Aufgabenstellung und die Art der Anwendung. Die in Kultur gezüchteten Zellgewebe werden auf die äussere Körperoberfläche als Hautersatz aufgebracht. Bei vorliegendem Verfahren werden jedoch die gezüchteten Zellen in vitro auf alloplastischen oder devitalisierten biologischen Materialien, wie auch auf Organtransplantaten als Zellrasen aufgezüchtet und andererseits für die Konditionierung des Empfängerorganismus verwendet. Die Implantate bzw. Transplantate werden bevorzugt ins Körperinnere implantiert, und es werden allogene oder xenogene Spenderzellen verwendet, die bei der Zellzüchtung durch Inkompatibilitätsfaktoren aus dem Empfängerorganismus konditioniert werden. Bei der erwähnten Literaturstelle werden zur Abdeckung von Verbrennungen ausschliesslich autologe Gewebe verwendet, sodass diese konditionierende Vorbereitung der Zellen und des Empfängerorganismus nicht bekannt war.

Die Verwendung von vorbereiteten Implantaten mit aufgezüchteten allogenen, xenogenen oder foetalen Geweben und Zellen, bietet trotz der erforderlichen Vorbereitung des Empfängers den Vorteil, einer kürzeren Vorbereitungszeit als bei der Anzüchtung von autologem Gewebe. Auch wurde in der angeführten Literaturstelle ausschliesslich Hautepithel verwendet, das sich von den im vorliegenden Verfahren verwendeten Zellarten grundsätzlich unterscheidet.

Die alloplastischen Implantate mit aufgezüchtetem Zellrasen werden in Art der Vitalkonservierung bei der Gewebezüchtung in einem Nährmedium bei Temperaturen zwischen + 5° und + 35°C gehalten, wobei eine Benetzung der aufgezüchteten Zellen und der Schutz vor mechanischer Alteration gewährleistet sein muss. Ein stufenweises Einfrieren ist bei Verwendung von Vitrikationsflüssigkeiten z.B. Dimethylsulfoxyd oder Glyzerin möglich. Das Auftauen erfolgt wie bei der Gewebe- und Zellkultivierung und erfordert eine anschliessende Konditionierung in Nährmedien mit den genannten wachstumsfördernden Stoffen. Organtransplantate können jedoch nicht kältekonserviert werden.

Weitere Merkmale und Vorteile von bevorzugten Ausführungsformen der Erfindung ergeben sich aus den nachfolgenden Beispielen.

3

Beispiel 1

Es sollen nach länger zurückliegendem Zahnverlust Zähne aus alloplastischen Materialien implantiert werden unter Mitverwendung von autologen Zellgeweben, die vorher auf dem Implantat aufgezüchtet werden.

Als Implantat werden vorgefertigte, sterile Materialien aus Biokeramik oder Biogläsern verwendet, die in Grösse und Form mit'dem vorgesehenen Implantatbett übereinstimmen. Mindestens 1 Woche vor der Implantation wird chirurgisch oder durch Nadelbiopsie Gewebe aus der späteren Implantationsstelle gewonnen, unter sterilen Kautelen mit physiologischen Salzlösungen (Tyrode-Lösung, Hanks-, Locke- oder Ringerlösung) gespült und in einem der bekannten natürlichen Nährmedien, z.B. nach Baker oder Lewis oder aber in einem künstlichen Nährmedium bekannter chemischer Zusammensetzung, z.B. nach Eagle, Earl, Evens, Nagel, Parker, White u.a. mit Zusatz einer geeigneten Antibiotikamischung, z.B. aus Aureomycin, Bacitracin, Chloramphenicol, Neomycin, Penicillin, Polymycin, Streptomycin, Terramycin bei Temperaturen zwischen $+5°$ und $+30°C$ zum Zellaboratorium gebracht. Dort wird nach bekannten Zellzüchtungsmethoden, die zu möglichst intensiver Zellproliferation führen, aus dem Explantat die Zellkultivierung durchgeführt. Es ist dabei möglich, durch wiederholtes Umsetzen der Kulturen, bestimmte Zellarten durch Klonen isoliert zu züchten und auch eine Massenzüchtung der Zellen durchzuführen.

Zur Stimulierung des Zellwachstums werden zytoplasmatische Organextrakte, wahlweise einzeln oder in Mischungen, aus foetaler Zahnleiste, Leber, foetaler Plazenta und Thymus, wie auch gegebenenfalls von anderen heterologen Organarten, z.B. vom Rind, in einer Konzentration von $10^{-4}$ bis $10^{-6}$ g Protein/ml Nährmedium bei den einzelnen Passagen zugesetzt. Es können dazu auch Stimulationsfaktoren verwendet werden, die nacn EPA 80 106 066.6 aus denselben Organarten gewonnen wurden oder andere bekannte, wachstumsfördernde und entzündungshemmende Extrakte, wie z.B. Carnosin (vgl. K. Nagai: Entzündungshemmung durch die Förderung der spontanen Heilung mittels L-Carnosin: Langenbecks Arch. Chir. 351, 39–39 (1980)), Epidermis-Wuchsfaktor (D. Gospodarowicz et al.: Anm. Rev. Biochem. 45 (1976) 531–558), Somatotropin u.a. Eine adaptive Anpassung der kultivierten Zellen an den späteren Empfänger bzw. die Toleranzerzeugung im Empfänger, ist bei Verwendung autologer Gewebe nicht erforderlich.

Aus einer Monolayer-Kultur wird mindestens 2–3 Tage vor der vorgesehenen Implantation der Zellrasen durch Trypsin-Versenlösung abgelöst oder durch Enzymeinwirkung eine Zellsuspension gewonnen, die von Enzymen freigespült, auf das vorgesehene Implantat an verschiedenen Stellen, die später mit dem Implantationsbett in Berührung kommen, aufgeimpft wird. Um das Aufwachsen des Zellrasens zu erleichtern, kann nach bekannten Methoden (vgl. B. Mauersberger: Zell- und Gewebezüchtung: G. Fischer Verlag, Stuttgart, 1977) die alloplastische Materialunterlage vor der Beimpfung der Zellsuspension oder dem Aufbringen eines bereits gezüchteten Zellrasens, mit Fibrin, Collagen oder Elastin beschichtet werden. Die beimpften Stellen des Implantates werden unter Kulturbedingungen entweder fortlaufend oder rhythmisch intermittierend mit Nährlösung befeuchtet. Zur rhythmischen Befeuchtung werden die Zahnexplantate z.B. an den Längsenden drehbar fixiert und in einer Wanne mit Medium eingetaucht. Die Umdrehung besorgt eine spezielle Apparatur. Nachdem der Zellrasen fest auf dem Implantat angewachsen ist, wird dieser in ein Transportgefäss übergeführt. In dem Transportgefäss muss die Befeuchtung der Zellen mit sauerstoffhaltigem Nährmedium zur Implantation gewährleistet sein. Es wird dazu Tyrode-Lösung mit Zusatz von 50–100 Einheiten Penicillin und Streptomycin pro ccm verwendet. Die Temperatur während des Transportes wird zwischen $+5°C$ und $+20°C$ gehalten. Gleichzeitig wird separat ein Teil der verwendeten Zellsuspension im Nährmedium mitgeliefert. Von dieser werden bei der Vorbereitung des Bioklebers aus Fibrin $2 \times 10^3$ Zellen/ccm in die Thrombinlösung eingemischt, die ihrerseits mit dem Fibrinogenkonzentrat zusammengemischt wird. Bei Erreichen des geeigneten Polymerisationsgrades des sich ausbildenden Fibrins, wird der Biokleber unmittelbar vor dem Implantat in das eröffnete Wundbett eingebracht. Zuvor kann man das Wundbett und das Implantat mit der Zellsuspension präparieren. Anstatt der Einbringung von suspendierten Zellen in den Biokleber oder zur vorherigen Benetzung des Implantates können auch wachstumsstimulierende Organextrakte oder Extrakte aus den kultivierten Zellen wie bei den Zellkulturen verwendet werden.

Der implantierte Zahn soll im Kieferknochen möglichst festsitzen. Bei der Implantation wird jede Bewegung des Implantates vermieden, die zu einer Abschiebung des Zellrasens führen könnte. Die Ränder des Wundbettes werden nach der Einpflanzung möglichst dicht am Implantat durch Nähte adaptiert und der aus der Wunde herausragende Teil des Implantates durch Schienenverband bis zum Festwerden des Implantates fixiert.

Beispiel 2

Für den Sofortersatz von Einzelzähnen werden Implantate mit aufgezüchteten, vitalkonservierten Zellen vorrätig gehalten. Es werden dabei Zellen aus foetaler, menschlicher Zahnleiste und bzw. oder aus Amnion, Nabelschnur und bzw. oder Plazenta aufgezüchtet. Zur Verbesserung des Einwachsens wird mindestens ½ Stunde vor der Implantation Blutserum vom Empfänger, gegebenenfalls in Mischung mit dem wässrigen Extrakt aus Homogenaten der weissen Blutzellen, der Nährlösung für die Aufzüchtung von Zellen auf dem Implantat zugesetzt. Das Wundbett, das Implantat sowie auch der Biokleber wird, wie in Beispiel 1, mit wachstumsstimulierenden und entzündungshemmenden Faktoren vorbereitet.

Beispiel 3

Die Verwendung von allogenen und xenogenen Zellkulturen erfordert eine konditionierende Vorbereitung der Zellen und möglichst auch des Empfängerorganismus. Gleichzeitig mit den in Beispiel 1 beschriebenen wachstumsstimulierenden Faktoren, werden Blutserum, Extrakte aus Zellhomogenaten von weissen Blutzellen und bzw. oder von Biopsiematerial vom späteren Empfängerorganismus einem Minimalnährmedium während 8 Stunden oder wenn möglich, während 1 oder 2 Passagen vor der Implantation zugesetzt. Danach wird das optimale Nährmedium mit wachstumsstimulierenden Faktoren ebenfalls mit diesen Zusätzen verwendet. Der vorgesehene Empfängerorganismus wird mit Histokompatibilitätsfaktoren aus dem Spenderorganismus oder aus den zur Transplantation vorgesehenen Zellkulturen, in Form von Einzelzellen oder Zellextrakten, während mindestens 3 Tagen vor der Implantation, in steigender Dosierung, wiederholt vorbehandelt. Es werden dazu besondere Zell- bzw. Gewebspresssäfte aus den kultivierten Zellen und bzw. oder das dekantierte Kulturmedium parenteral oder oral, in sich verlängernden zeitlichen Abständen, zunächst halbstündlich, dann 3–2mal täglich, sodann in täglichen Abständen vor der Implantation verabreicht. Auch können Suspensionen der gezüchteten Zellkulturen in ansteigender Zellzahl, z.B. beginnend mit $10^2$-, dann $10^4$- bis $10^6$-Zellen/ccm zusammen mit Verdünnungen der Kulturflüssigkeit in ng, µg, mg Protein/ccm in physiologischer Lösung appliziert werden.

Beispiel 4

Konditionierung von Transplantat und Empfängerorganismus für die Organtransplantation einer allogenen, nicht vollkompatiblen Niere:

Vom lebenden Organspender werden mindestens 8 Tage vor der Nierenspende, wie in Beispiel 3, Histokompatibilitätsantigene aus Zellgeweben gewonnen. Insbesondere werden aus leukozytären Blutzellen bzw. aus Biopsiematerial Zellkulturen angelegt. Zur Erzeugung von Immuntoleranz des Empfängers gegenüber dem Transplantat, werden Blutserum des Spenders, Zellextrakte aus den gezüchteten Zellen oder lebende Zellsuspensionen in ansteigender Dosierung während mindestens 3 Tagen vor der Transplantation wiederholt in ansteigender Dosierung und sich verlängernden Intervallen dem Empfänger appliziert. Diese tolerogene Vorbehandlung des Empfängers gegen das Transplantat kann gleichzeitig kombiniert mit der Vorbehandlung gegen die darauf aufgezüchteten Zellen, falls diese nicht vom selben Spender wie das Transplantat stammen, vorgenommen werden.

Bei Verwendung von Leichenorganen wird das Organ nach bekannten Verfahren der Organtransplantation durch vitale Konservierung am Leben erhalten (vgl. K.F. Bauer: Methodik der Zell- und Gewebezüchtung – über die Organexplantation). Gleichzeitig werden Zellexplantate kultiviert und daraus Zellextrakte mit Histokompatibilitätsfaktoren gewonnen. Es können dazu Blutpräparate (Serum, Blutzellen) vom Spenderorganismus mitverwendet werden. Der Empfängerorganismus wird während mindestens 4–8 Tagen vor der Organtransplantation damit vorbehandelt. Andererseits wird während dieser Zeit dem Nährmedium für das Organexplantat von aussen, wie auch von innen durch Anschluss an das Gefässsystem des Explantates, Nährlösung mit Zusatz dieser Histokompatibilitätsfaktoren vom späteren Empfänger, in ansteigenden Konzentrationen über ng, µg, mg Eiweissgehalt/ml Medium, in sich verlängernden Abständen von ½ Stunde bis 6–8 Stunden, mehrmals zugesetzt. Gleichzeitig werden auch hier wachstumsstimulierende Faktoren, wie in Beispiel 1, mitverwendet.

Für die Verwendung von allogenen und insbesondere xenogenen Eihäuten oder Nabelschnur in der Chirurgie, ist eine immunologisch-tolerogene Vorbehandlung des Empfängerorganismus, mit Zellen oder Zellextrakten aus den zu überpflanzenden Geweben zweckmässig. Die serösen Häute werden entsprechend dem vorhergehenden Beispiel, während der Kultivierung an den Empfänger oder an allogene Histokompatibilitätsfaktoren aus dem Empfängerorganismus, adaptiert.

Das Aufzüchten von autologen oder an den Empfänger adaptierten Zellen auf das Organtransplantat erfolgt, wie bei alloplastischen Implantaten. Es werden dazu möglichst foetale Zellen, nach wenigen Zellteilungen, verwendet. Eine Konditionierung dieser Kulturen wird sowohl mit Gewebefaktoren vom späteren Empfängerorganismus, wie auch mit solchen aus dem Spenderorganismus der Organtransplantate oder den daraus kultivierten Zellen, durchgeführt. Auch die Transplantation erfolgt in ähnlicher Weise unter Berücksichtigung spezieller chirurgischer Techniken. Das Wundbett wird nach vollkommener Blutstillung ebenfalls mit wachstumsstimulierenden Extrakten und Faktoren vor der Organüberpflanzung präpariert. Auch können Biokleber mit wachstumsfördernden und synthesestimulierenden Eigenschaften verwendet werden.

Beispiel 5

Die Vorbereitung einer Gelenks-Endoprothese, z.B. des Knie- oder Hüftgelenkes, erfolgt grundsätzlich in gleicher Weise wie die Implantation von Zähnen. Es werden hier bevorzugt kultivierte Osteoblasten und Fibroblasten, möglichst aus Gewebeexplantaten vom Empfängerorganismus oder aber allogenen Ursprungs an den Stellen aufgezüchtet, die im Knochen fixiert werden sollen. Es werden dazu auch foetale, allogene Zellen aus Gelenken, Gelenkkapsel und Knochen verwendet, die an den späteren Empfängerorganismus durch Vorbehandlung mit Histokompatibilitätsfaktoren vom Empfänger, in Form von Zellextrakten aus Blutzellen oder Biopsiematerial, adaptiert sind. Bei Verwendung von allogenem oder xenogenem Zellmaterial wird der Empfänger entsprechend den vorherigen Beispielen immunologisch tolerogen vorbehandelt.

Beispiel 6

Die Transplantation von devitalen Geweben erfolgt in gleicher Weise wie in Beispiel 1, durch Aufwachsen eines vom Empfängerorganismus tolerierten Zellrasens, der gleichzeitig wie im Beispiel 5, an das Transplantat adaptiert ist.

Beispiel 7

Kältekonservierung des mit Zellen beschichteten Implantates:

Unter Zusatz von Kälteschutzmitteln, z.B. 36% Ammoniumacetat, 39% Dimethylsulfoxyd oder 40% Glyzerin zur Nährlösung, erfolgt ein stufenweises Eingefrieren durch Abkühlung von − 1° bis − 2°C pro Minute bis mindestens − 90°C, möglichst bis − 196°C. Bei dieser Temperatur bleiben die Zellgewebe für fast unbegrenzte Zeit revitalisierbar. Die Auftauung erfolgt ebenfalls nach bekannten Methoden der Gewebe- und Zellzüchtung (vgl. K.F. Bauer) entweder durch schlagartiges Erwärmen auf 37°C oder, je nach Thermoschockbelastbarkeit des Implantatmaterials durch stufenweises Erwärmen von 0,3° bis 3°C pro Minute. Nach Auftauung ist wie bei der Routinezüchtung der Zell- und Gewebekulturen weiter zu verfahren.

**Patentansprüche**

1. Verfahren zur Vorbereitung von Implantationen, wobei alloplastische oder devitalisierte biologische Materialien für die Rekonstruktion von Gewebedefekten an Knochen, an Sehnen, an Blutgefässen, an Haut und an serösen Häuten sowie für den Ersatz von Zähnen, Gelenken (Endoprothesen) und Sehnen, insbesondere aus devitalisierten Knochen, Knochenspänen und -splittern, Sehnen, aus Kunststoffen, Keramik, Biogläsern und anderen geeigneten Materialien und aus Kombinationen verschiedener biologisch-chemisch inerter, zur Implantation geeigneter Materialien benutzt werden, sowie Verfahren zur Vorbereitung von Transplantationen, wobei allogene oder xenogene, lebende Gewebe benutzt werden, dadurch gekennzeichnet, dass man die Oberfläche der alloplastischen Materialien bzw. der allogenen oder xenogenen Gewebe beschichtet durch An- bzw. Einwachsen eines Zellrasens von in Kultur gezüchteten, einem Spender entnommenen lebenden Gewebezellen, dass man aus den Zellen des Spenders oder daraus hergestellten Zellextrakten und bzw. oder dekantierten Nährmedien, in denen die Zellen gezüchtet werden, Präparate zur tolerogenen Vorbehandlung des Empfängers herstellt, wobei man bei der Züchtung der Spender-Zellen Histokompatibilitätsfaktoren aus dem späteren Empfänger, sowie Gewebe-stimulierende Faktoren, insbesondere aus foetalen und jugendlichen Organgeweben, den Zellkulturen zusetzt, und dass man Biokleber für die Transplantation bzw. Implantation bereitstellt, die für Körperflüssigkeiten diffusibel sind und die entsprechende Spender-Zellen oder daraus gewonnene Zellextrakte und andere Wachstums-fördernde Faktoren enthalten können.

2. Verfahren nach Anspruch 1, dadurch gekennzeichnet, dass man sterile, alloplastische Materialien mit netz- oder schwammartig durchbrochener Struktur oder aufgerauhter bzw. feinporöser Oberfläche zur Aufzüchtung des Zellrasens verwendet.

3. Verfahren nach Anspruch 1, dadurch gekennzeichnet, dass man extrahierte Zähne oder Biopsiematerial aus der vorgesehenen Implantationsstelle des Empfängers zur Herstellung der Gewebezellen bzw. Gewebekulturen verwendet.

4. Verfahren nach Anspruch 1, dadurch gekennzeichnet, dass, die aus dem Spender in vitro gezüchteten Zellen insbesondere Osteoblasten, Odontoblasten, Chondroblasten, Myoblasten, Fibroblasten oder epitheliale Zellen sind und auf dem vorgesehenen Implantat an verschiedenen Stellen angeimpft und dauernd oder rhythmisch mit geeignetem Nährmedium nach bewährten Methoden der Zellzüchtung befeuchtet werden, gegebenenfalls unter Verwendung besonderer mechanischer Einrichtungen für das kontinuierliche Drehen des späteren Implantates im Nährmedium.

5. Verfahren nach Anspruch 1, dadurch gekennzeichnet, dass die Vitalkonservierung des hergestellten Implantates bzw. Transplantates in einem geeigneten Nährmedium bei Temperaturen zwischen + 5° und + 35°C oder in tiefgefrorenem Zustand nach den bekannten schonenden Methoden der Gewebezüchtung und Gewebekonservierung, gegebenenfalls unter Zusatz von Vitrikationsflüssigkeiten, erfolgt.

6. Verfahren nach Anspruch 1–4, dadurch gekennzeichnet, dass als Spenderorgan foetale Gewebe, Haut, Eihäute, Nabelstrang, Plazenta, Thymus, Blutadern, Knorpel, Gelenkkapsel, Knochenmark oder Zahnleiste verwendet werden.

7. Verfahren nach Anspruch 1–6, dadurch gekennzeichnet, dass zur Adaptation der Spender-Zellen an den Empfänger den Nährmedien wachstumsstimulierende Stoffe oder Differenzierungsstoffe oder allogene oder xenogene Organextrakte bzw. -hydrolysate, insbesondere Gewebeextrakte aus Körper- oder Blutzellen bzw. -serum des späteren Empfängers, zugesetzt werden.

8. Verfahren nach Anspruch 7, dadurch gekennzeichnet, dass Stimulationsfaktoren, die nach dem Verfahren zur Gewinnung von biologischen Wirkstoffen nach EPA Nr. 80 106 066.6 gewonnen sind, zur Zellzüchtung und bei der Implantation verwendet werden.

9. Verfahren nach Anspruch 1, dadurch gekennzeichnet, dass dem Biokleber für die Implantation bzw. Transplantation Wachstums- und Differenzierungsfaktoren aus Gewebezellen oder Suspensionen der gezüchteten Zellen zu der flüssigen Phase eines Gels oder zu einem Fibrinogenkonzentrat, zu einer Thrombinlösung oder zu möglichen anderen Bioklebern, die auch zur Imprägnation des Implantates bzw. des Implantationsbettes verwendet werden, zugegeben werden.

10. Verfahren zur Herstellung von Präparaten zur Erzeugung von Immuntoleranz gegenüber histoinkompatiblen Gewebebestandteilen des zugehörigen Spenders in einem Empfänger eines Organtransplantates, dadurch gekennzeichnet, dass diese Präparate aus dem Spender des Organtransplantates gewonnen werden.

**Claims**

1. Method for the preparation of grafts, in that alloplastic or devitalized biological material is used for the reconstruction of tissue defects on bones, tendons, blood vessels, skin and on serous skins as well as for the replacement of teeth, joints (endoprothesis) and tendons, in particular from devitalised bones, bone chips and splinters, tendons, from plastic, ceramic, biological glass and other suitable materials and from combinations of various biologic-chemically inert materials, suitable for implantation (grafting), as well as method for the preparation of transplantations (grafts), in that allogenous or xenogenous, living tissue is used, characterized in that, the alloplastic material surface or the allogenous or xenogenous tissue respectively are coated by surface growth or growing in of a cell substrate cultivated from living tissue cells taken from a donor, preparations for the tolerogenous pretreatment of the recipient are produced from the donor's cells or from cell extracts made of the latter cells or from decanted nutritive substance, by adding to the cell cultures, when culturing donor cells, histocompatibility factors of the intended recipient, as well as tissue stimulating factors, originating in particular from foetal and juvenile organ tissues, bioadhesive for the transplantation or implantation is made available which the body fluids are unable to diffuse and which can contain the corresponding donor cells or the cell extracts of same and other growth causing factors.

2. Method in accordance with claim 1, characterized in that sterile alloplastic materials with lattice or sponge type perforated structures or roughened or fine porous surfaces are used for culturing the cell substrate.

3. Method in accordance with claim 1, characterized in that extracted teeth or biopsy material from the recipient's planned implant (graft) position is used for the production of tissue cells or tissue cultures.

4. Method in accordance with claim 1, characterized in that the cells grown from the donor in vitro, in particular are osteoblasts, odontoblasts, chondroblasts, myoblasts, fibroblasts or epithelial cells vaccinated at various points on the intended implant (graft) and moistened continuously or rhythmically with a suitable nutritive medium in accordance with the proven methods of cell cultivating, if necessary by using special mechanical devices assuring continuous rotation of the later implant (graft) in the nutritive medium.

5. Method in accordance with claim 1, characterized in that the vital conservation of the produced implant (graft) or transplant is made in a suitable nutritive medium at a temperature between +5° and +35°C or in a deep frozen condition in accordance with the known gentle and careful method of tissue culture and tissue conservation, if necessary by adding vitrication fluids.

6. Method in accordance with claims 1–4, characterized in that foetal tissue, skin, egg skin, umbilical cord, placenta, thymus gland, blood vessels, cartilage, joint capsule, bone marrow or dental ridge are employed.

7. Method in accordance with claims 1–6, characterized in that, for the adaption of the donor cell to the recipient growth stimulating material or differentiation material or allogenous or xenogenous organ extracts or hydrolysates, in particular tissue extract from body or blood cells or serum of the later recipient are added to the nutritive media.

8. Method in accordance with claim 7, characterized in that the stimulating factors obtained in accordance with the method for obtaining biologically active principles as being stipulated in EPA No. 80 106 066.6, are used for cell cultivation and grafting.

9. Method in accordance with claim 1, characterized in that for implantation or transplantation the bioadhesive is added growth and differentiation factors from tissue cells or suspensions of the cultivated cells to the liquid phase of a gel or to a fibrinogen concentrate, to a thrombin solution or to permit other bioadhesives, which are also used for implants or grafting substrate impregnation.

10. Method for the manufacture of preparations for creating immunity tolerance to histo incompatible tissue components of the attendant donor in the organ transplant recipient, characterized in that these preparations are obtained from the organ transplant donor.

**Revendications**

1. Procédé pour la préparation d'implantations dans lequel sont utilisées des matières alloplastiques ou des matières dévitalisées biologiques pour la reconstruction de défauts tissulaires aux os, tendons, vaisseaux sanguins, à la peau et aux peaux séreuses, ainsi que pour le remplacement de dents, articulations (endoprothèses) et tendons, provenant en particulier d'os dévitalisés, copeaux d'os et esquilles, tendons, de matières plastiques, céramique, biocristaux et autres matières appropriées et de combinaisons de plusieurs matières inertes du point de vue biologicochimique et appropriées pour des implantations, ainsi que des procédés pour la préparation de transplantations pour lesquels sont utilisés des tissus vivants allogènes ou xénogènes, caractérisé par le fait que l'on revêt la surface des matières alloplastiques resp. des tissus allogènes ou xénogènes d'une pelouse greffée de cellules tissulaires vivantes prélevées sur un donneur et mises dans une culture; que l'on obtient des cellules du donneur ou des extraits de celles-ci et/ou des bouillons de culture décantés, dans lesquels les cellules sont cultivées, des préparations pour le prétraitement tolérogène du receveur tout en ajoutant aux cultures des cellules du donneur des facteurs histocompatibles venant du receveur, ainsi que des facteurs stimulant les tissus provenant en particulier de tissus organique foetaux ou juvéniles; et que l'on tient à disposition pour la transplantation resp. l'implantation des colles biologiques diffusibles pour les liqueurs corporelles et pouvant contenir les cellules du donneur ou les extraits faits de celles-ci ou d'autres facteurs favorisant la croissance.

2. Procédé selon spécification 1, caractérisé par le fait que l'on utilise des matières stériles alloplastiques d'une structure à percées réticulées ou spongeuses ou avec une surface rugueuse resp. aux micropores pour cultiver la pelouse cellulaire.

3. Procédé selon spécification 1, caractérisé par le fait que l'on utilise pour obtenir des cellules tissulaires resp. des cultures tissulaires, des dents arrachées ou des matières biopsiques du receveur.

4. Procédé selon spécification 1, caractérisé par le fait que les cellules du donneur, cultivées in vitro, sont en particulier d'ostéoblastes, odontoblastes, chondroblastes, myoblastes, fibroblastes ou des cellules épithéliales, qui sont greffées à divers points sur l'implant prévu et qui sont en permanence ou de manière rythmique humectées avec un bouillon de culture approprié d'après des méthodes éprouvées de la culture cellulaire; le cas échéant en utilisant des installations mécaniques particulières pour retourner de façon continue l'implant prévu dans le bouillon de culture.

5. Procédé selon la spécification 1, caractérisé par le fait que la conservation vitale de l'implant resp. du greffon obtenu se fait dans un bouillon de culture approprié à une température entre +5 et +35°C ou congelé d'après des méthodes connues de la culture et de la conservation tissulaire, le cas échéant en y ajoutant des liquides vitrifiants.

6. Procédé selon les spécifications 1–4, caractérisé par le fait que sont utilisés comme organe du donneur des tissus: peau, membranes ovulaires, cordon ombilical, placenta, thymus, veines, cartilage, capsules articulaires, moelle osseuse ou lame dentaire.

7. Procédé selon les spécifications 1–6, caractérisé par le fait que pour adapter les cellules du donneur au receveur sont ajoutées aux bouillons de culture des substances stimulant la croissance ou des substances de différenciation ou des extraits resp. d'hydrolysats d'organes allogènes ou xénogènes, en particulier des extraits tissulaires des cellules du corps ou du sang resp. du sérum sanguin du futur receveur.

8. Procédé selon la spécification 7, caractérisé par le fait que sont utilisés des facteurs stimulants, lesquels sont obtenus d'après le procédé pour l'obtention de biocatalyseurs d'après EPA No. 80 106 066.6 pour la culture des cellules et l'implantation.

9. Procédé selon la spécification 1, caractérisé par le fait que sont ajoutés à la colle biologique pour l'implantation resp. transplantation des facteurs de croissance ou de différentiation provenant de cellules tissulaires ou de suspensions de cellules cultivées mêlés à la phase liquide d'un gel ou un concentré fibrinogène, une solution de thrombine ou d'autres colles biologiques possibles, qui sont utilisées pour impregner l'implant ou le lit de l'implant.

10. Procédé pour l'obtention de préparations engendrant la tolérance immunitaire contre les substances tissulaires histocompatibles du donneur dans le receveur d'un transplant d'organe, caractérisé par le fait que ces préparations sont obtenues du donneur du transplant.